# EUROPÄISCHE PATENTSCHRIFT

(11) **EP 3 554 956 B1**
(45) Veröffentlichungstag und Bekanntmachung des Hinweises auf die Patenterteilung: **13.05.2020**
(21) Anmeldenummer: 19708839.6
(22) Anmeldetag: 01.03.2019
(51) Int. Cl.: B65D 25/28, A61L 2/26, A61B 50/20, A61B 50/30

(54) **STERILISIERSIEBSCHALE MIT EINEM MITTELS EINER BETÄTIGUNG IN DIE ENTNAHMEPOSITION ÜBERFÜHRBAREN GRIFF**
STERILISABLE STRAINER DISH COMPRISING A HANDLE THAT CAN BE SHIFTED INTO THE REMOVAL POSITION BY MEANS OF ACTUATION
COQUE FILTRANTE DE STÉRILISATION POURVUE D'UNE POIGNÉE POUVANT ÊTRE AMENÉE DANS LA POSITION DE PRÉLÈVEMENT AU MOYEN D'UN ACTIONNEMENT

(30) Priorität: 05.03.2018 DE 102018104942
(43) Veröffentlichungstag der Anmeldung: 23.10.2019
(73) Patentinhaber: Aesculap AG, 78532 Tuttlingen (DE)
(72) Erfinder: GÖRZ, Dennis, 78532 Tuttlingen (DE); ROSIN, Bianca, 78532 Tuttlingen (DE); STREIT, Eva, 78351 Bodman-Ludwigshafen (DE); KNITTEL, Timo, 78573 Wurmlingen (DE); WELLER, Frank, 23879 Mölln (DE); HENKE, Matthias Dr., 78567 Fridingen (DE)
(74) Vertreter: Winter, Brandl, Fürniss, Hübner, Röss, Kaiser, Polte - Partnerschaft mbB
(86) Internationale Anmeldenummer: PCT/EP2019/055184
(87) Internationale Veröffentlichungsnummer: WO 2019/170552

(56) Entgegenhaltungen:
- DE-A1-102006 062 379
- DE-C1- 10 101 424
- DE-U1-202010 005 788
- US-A- 5 005 255

## Beschreibung

Die vorliegende Erfindung betrifft eine Sterilisiersiebschale, auch Siebkorb genannt, etwa einen Sterilisations- oder Desinfektionssiebkorb, gemäß dem Oberbegriff des Anspruchs 1. Gattungsgemäße Siebkörbe werden eingesetzt, um in einem Reinigungs- und Desinfektionsgerät (RDG) oder Autoklaven in einer Aufbereitungseinheit für Medizinprodukte (AEMP) bzw. einer zentralen Sterilgutversorgungsabteilung (ZSVA) einen tragbaren Aufnahmebehälter für eine Anzahl zu desinfizierender oder sterilisierender Gegenstände, etwa chirurgische Schneid- oder Greifinstrumente, bereitzustellen.

### Hintergrund der Erfindung

Siebkörbe sind Teil des Instrumentenkreislaufs und werden immer wieder zyklisch gereinigt und mit (etwa chirurgischen) Instrumenten bepackt, bevor sie in beispielsweise Sterilisationsbehältern / Sterilcontainern sterilisiert werden und ausgehend von der AEMP in einen Operationssaal (OP) transportiert werden.

Vor diesem Hintergrund sollten Siebkörbe leicht transportierbar bzw. handhabbar sein und folglich Griffe aufweisen. Jene Griffe sollten zum ersten jedoch nicht bzw. möglichst wenig das von dem Siebkorb aufgespannte Packvolumen beeinträchtigen/beschränken. Hieraus resultiert, dass die Griffe der Siebkörbe möglichst wenig bis gar keinen Platz im Innenraum des Siebkorbs einnehmen sollten. Zum zweiten sollten die Griffe den Beladungs- bzw. Packprozess (mit Instrumenten) möglichst wenig behindern, während sie zeitgleich von einem Anwender gut erreichbar sein sollten.

Letztlich sollten die Griffe zum dritten auch derart ausgestaltet sein, dass im OP eine aseptische / keimfreie Entnahme der beladenen Instrumente möglichst ergonomisch möglich ist.

### Stand der Technik

Aus der DE 000010101424 C1 ist etwa ein Siebkorb mit einem Fall-/Klappgriff bekannt. Dieser Griff kann eine Entnahmeposition einnehmen, in der er komfortabel greifbar aus dem Innenraum des Siebkorbs hervorsteht, um ein Tragen des Siebkorbs zwischen den einzelnen Stationen seines Zyklus zu ermöglichen. Des Weiteren kann der Griff eine Verriegelungsposition einnehmen, in der ein Beladen des Siebkorbs möglich ist. Die DE 000010101424 C1 widmet sich bereits der Herausforderung, den Griff in der Entnahmeposition leicht zu erreichen und ihn in der Verriegelungsposition möglichst platzsparend im Innenraum zu lagern.

Nachteilig an dem Fall-/Klappgriff aus dem Stand der Technik ist, dass der Griffe bzw. die Griffe in der Verriegelungsposition tief in den Innenraum hineinragen, sodass der von ihnen eingenommene Platz im Innenraum nicht mehr von zu beladenden Gegenständen / Instrumenten eingenommen werden kann. Das Beladevolumen des Siebkorbs verringert sich also.

Darüber hinaus bergen jene Fall-/Klappgriffe den Nachteil, dass sie bei einer aseptischen Entnahme, bei der möglichst wenige Flächen des Siebkorbs mit der Umgebung in Berührung zu bringen sind, sehr unhandlich, d.h. "nicht-ergonomisch", aus dem Siebkorb zu führen sind bzw. nur unter erhöhtem Aufwand von der Verriegelungsposition in die Entnahmeposition überführbar sind: Entweder der Griff ist mit dem Daumen eines Anwenders hochzuziehen (was leicht zu einem Verrutschen des Griffs führt) oder die Hände sind so zu drehen, dass die Handinnenfläche nach außen / lateral zeigt (was unergonomisch ist).

Weiterhin nachteilig an den Fall-/Klappgriffen ist, dass sie beim Hochziehen der Fallgriffe unerwünschterweise Instrumente, mit denen der Siebkorb beladen ist, mit sich führen können, sodass diese angehoben und unter Umständen sogar beschädigt und/oder verschmutzt werden.

Aus der DE 102006062379 A1 ist ein Sterilbehälter mit beweglich gelagerten Tragegriffen bekannt. Die US 5005255 offenbart Griffe mit einer Verriegelungsposition.

### Kurzbeschreibung der Erfindung

Der vorliegenden Erfindung liegt angesichts dieses Standes der Technik die Aufgabe zugrunde, die Nachteile aus dem Stand der Technik zu beheben oder zumindest zu mildern, und insbesondere einen Siebkorb zur Verfügung zu stellen, der solche Griffe aufweist, die zum einen in der AEMP möglichst wenig (bis gar keinen) Platz im Innenraum des Siebkorbs einnehmen und den Pack-Prozess / das Beladen mit Instrumenten möglichst wenig (bis gar nicht) beeinträchtigen. Zum anderen soll ein solcher Griff bereitgestellt werden, der im OP ein ergonomisches, aseptisches Überführen von der Verriegelungsposition in die Entnahmeposition ohne (großen) zusätzlichen Aufwand ermöglicht und dementsprechend auch ein ergonomisches, aseptisches Entnehmen des Siebkorbs aus dem Sterilisationsbehälter gewährleistet.

Dies wird erfindungsgemäß mittels eines Siebkorbs mit den Merkmalen des Anspruchs 1 gelöst. Vorteilhafte Ausführungsformen sind Gegenstand der Unteransprüche.

Der Grundgedanke bzw. das Funktionskonzept der vorliegenden Erfindung besteht im Wesentlichen darin, den vorstehend beschriebenen, bisher vollständig manuell ausgeführten Überführungsvorgang zum Überführen/Verbringen des Griffs, bzw. dessen Halte-/Greifbügel aus seiner Verriegelungsposition in die Entnahmeposition gemäß vorstehender Definitionen funktional sowie vorzugsweise konstruktiv in eine manuell zu aktuierende Halte-/Greifbügel - Freigabefunktion und eine nachfolgende automatisch/selbsttätig aktuierte Überführfunktion funktional sowie vorzugsweise konstruktiv zu trennen. Diese funktionale und ggf. konstruktive Zweiteilung des gesamten Überführungsvorgangs erlaubt es prinzipiell, den Griff bzw. dessen Halte-/Greifbügel derart am Siebkorb kompromisslos platzsparend anzuordnen, dass er bei Einnehmen seiner Verriegelungsposition überhaupt nicht mehr oder nur noch schwer manuell unmittelbar erreichbar/aktuierbar ist. Stattdessen ist erfindungsgemäß ein (separater) Freigabe-/Betätigungsmechanismus (zusätzlich zu dem Halte-/Greifbügel) am Siebkorb von außerhalb des Siebkorbs (frei) zugänglich angeordnet, welcher lediglich dafür vorgesehen und ausgebildet ist, den Halte-/Greifbügel in seiner Verriegelungsposition zu halten und bei dessen manueller Aktuierung den Halte-/Greifbügel freizugeben. Darüber hinaus ist der Griff mit einem Antriebsmechanismus versehen bzw. diesem zu-/beigeordnet, der dafür vorgesehen und ausgebildet ist, dessen Halte-/Greifbügel selbsttätig/automatisch aus seiner Verriegelungsposition in seine Entnahmeposition zu verbringen (nachdem der bzw. deren Halte-/Greifbügel manuell für eine Freigabe aktuiert worden ist).

Aus dieser erfindungsgemäßen Ausgestaltung eines Siebkorbs lassen sich somit beispielsweise folgende weitere Vorteile ableiten:
- Die Griffe bzw. deren Halte-/Greifbügel können nahezu beliebig so angeordnet sein/werden, dass sie eine möglichst platzsparende Verriegelungsposition einnehmen, die sich nicht/wenig auf den erreichbaren Kompaktheitsgrad der Beladung auswirken bzw. das nahezu maximale Beladevolumen erhalten.
- Ein Entnehmen des Siebkorbs aus dem Sterilisationsbehälter ist zeiteffizient und intuitiv möglich, da nicht mehr der Griff bzw. dessen Halte-/Greifbügel selbst unmittelbar manuell betätigt werden muss sondern lediglich ein separat hierzu vorgesehener, auf den Halte-/Greifbügel einwirkender Freigabe-/Betätigungsmechanism us.
- Ein Verrutschen oder sogar Beschädigen der beladenen Instrumente beim Überführen des Griffs bzw. dessen Halte-/Greifbügel von der Verriegelungsposition in die Entnahmeposition ist ausgeschlossen.
- Ein robustes Tragen und Bewegen des Siebkorbs ist durch stabil gelagerte Griffe ermöglicht.

Der Gegenstand der Erfindung ist demnach ein Siebkorb zur Aufnahme zu desinfizierender oder sterilisierender (allgemein: zu reinigender) medizinischer Gegenstände, mit einem Aufnahmebehälter und zumindest einem darin/daran gelagerten/drehbar angeordneten Griff bzw. dessen Halte-/Greifbügel, der in einer Entnahmeposition aus dem Aufnahmebehälter greifbar hervorsteht, um ein Tragen des Siebkorbs, etwa zum Entnehmen aus einem Sterilisationsbehälter, zu ermöglichen, und welcher in einer Verriegelungsposition vorzugsweise vollständig oder zumindest im Wesentlichen vollständig in dem Aufnahmebehälter und/oder nahe an dessen Behälterwand angeordnet ist, um ein freies, unversperrtes Be-/Entladen des Siebkorbs oder auch ein Transportieren des Siebkorbs in dem Sterilisationsbehälter zu ermöglichen.

Gemäß der Erfindung ist der Griff mittels der Betätigung eines erreichbaren, manuell ausrückbaren Halteelements des Freigabe-/Betätigungsmechanismus durch den dann zur Wirkung kommenden Antriebsmechanismus vorzugsweise vorgespannt, insbesondere federvorgespannt, von der Verriegelungsposition in die Entnahmeposition überführt bzw. überführbar. So ist das manuell ausrückbare Halteelement von einem Anwender in einem ersten Schritt betätigbar (da es von der Siebkorbaußenseite / von außen erreichbar / zugänglich ist), bevor, von diesem ersten Schritt ausgelöst / initiiert in einem zweiten Schritt der Griff von einer Position vorzugsweise im Inneren des Siebkorbs, d.h. von der Verriegelungsposition, in eine von außen zugängliche Position, d.h. in die Entnahmeposition, durch den Antriebsmechanismus bewegt wird. Hinter dem Mechanismus verbirgt sich eine ausgereifte Technik.

Dadurch ist es möglich, dass die beiden (scheinbar gegensätzlichen) Anforderungen der verbesserten Tragbarkeit bei optimierter Innenraumausnutzung jeweils für sich und somit synergistisch von der Erfindung erfüllt werden.

In anderen Worten lässt sich die Erfindung funktional derart beschreiben, dass an dem Siebkorb ein manuell ausrückbares Halteelement angebracht ist, dessen Betätigen ein dann automatisches Verfahren des Griffs von der Verriegelungsposition in die Entnahmeposition ermöglicht. Jene Automatik (Antriebsmechanismus) wird bevorzugt durch eine Feder, deren Vorspannung von der Betätigung des Halteelements freigesetzt wird, bewirkt. Anstelle eines eigenhändigen Verschwenkens gemäß dem Stand der Technik hat ein Anwender bei dem erfindungsgemäßen Siebkorb nur noch etwa eine Druck-/Zugbewegung am Halteelement, beispielsweise Bolzen, Kralle, Pratze, etc. durchzuführen, um den Haltebügel des Griffs auszufahren/auszuschwenken und den Siebkorb komfortabel aus dem Sterilisationsbehälter zu entnehmen. Besonders bevorzugt, da angenehm für den Anwender sowie platzsparend, ist das im weiteren Verlauf beschriebene Push-to-open-System / der Push-to-open-Mechanismus.

In einer vorteilhaften Ausführungsform ist zumindest eine Schenkelfeder für die Überführungsbewegung des Griffs von der Verriegelungsposition in die Entnahmeposition verantwortlich. Weiter bevorzugt ist an jedem Gelenk des Griffs, welches den Griff-/Haltebügel drehbar mit dem Aufnahmebehälter koppelt, eine (Griffeigene) Schenkelfeder angeordnet, sodass pro Griff zwei Federn eingesetzt werden, die eine robuste Führung / eine robuste Automatik des Griffs/Haltebügles beim Überführen von der Verriegelungsposition in die Entnahmeposition garantieren. Schenkelfedern zeichnen sich durch eine günstige Beschaffung sowie eine zuverlässige Wirkungsweise und einen geringen Bauraumbedarf aus.

Bevorzugt weist der Aufnahmebehälter des Siebkorbs eine im Wesentlichen rechteckige Grundform (Bodenplatte) auf, woraus bei ebenen (Vertikal-)Wänden ein quaderförmiger Aufnahmebehälter resultiert. Hierbei sind bevorzugt im Bereich der beiden Schmal-/Stirnseiten der Grundform / an den beiden schmalen (Vertikal-) Wänden des quaderförmigen Aufnahmebehälters jeweils ein bügelartiger Griff (aus einem flachen Stahl- oder Aluminiumband) angeordnet, der sich über die gesamte Stirnseite erstreckt. Diese bandartige, breite (d.h. über die nahezu gesamte Behälterschmalseite verlaufende) Ausgestaltung des Griffs ermöglicht ein stabiles Tragen / Entnehmen des Siebkorbs. Weiterhin sind in dieser Ausführungsform in den breiten Seitenwänden (d.h. nicht in den schmalen Stirnseiten) nahe der schmalen Seitenwände sich gegenüberliegende Gelenke / Lagerungen für den bügelartigen Griff vorhanden, was eine platzsparende Anordnung der Griffe weiter begünstigt.

In dieser Ausführungsform sind weiter vorteilhafterweise die bügelartigen Griffe in der Verriegelungsposition, vorzugsweise (annährend) schlitzfrei, entlang der schmalen Seitenwände angeordnet, sodass das von Gegenständen erreichbare Volumen des Innenraums des Aufnahmebehälters nur um das Volumen des jeweiligen bandartigen Griffs und somit minimal verkleinert wird. In anderen Worten ausgedrückt, schmiegt sich der Griffbügel an die Innenwand des Aufnahmebehälters an, sodass der Griffbügel kein "Totvolumen", das heißt ein Volumen, das von Gegenständen in der Bepackung nicht ausgefüllt werden kann, hinterlässt. Dies wirkt sich also günstig auf den verfügbaren Platz im Innenraum des Aufnahmevolumens aus.

In einer vorteilhaften Weiterentwicklung jener Ausführungsform ist der bügelartige Griff, der vorzugsweise in/an der breiten Seitenwand gelagert ist, weiter derart zumindest Längsabschnittsweise nach außen / lateral geschwungen, dass er bündig bzw. komplementär zur schmalen Seitenwand (Stirnfläche) verläuft und in der Verriegelungsposition einen Teil der Innenwand des Aufnahmevolumens selbst ausformt bzw. die schmale Seitenwand versteift. So nimmt der Griff in der Entnahmeposition (wie üblich) eine Grifffunktion ein und darüber hinaus nimmt er (erfindungsgemäß) in der Verriegelungsposition die Begrenzungs- und/oder Aussteifungsfunktion einer Innenwand ein. Somit ist das Volumen des Aufnahmebehälters durch den Griff (bis auf dessen Lagerung) nicht verkleinert, wodurch eine maximale Bepackung / Beladung möglich ist.

Wenn der Griff eine Begrenzungs-/Aussteifungsfunktion der Innenwand übernimmt, weist er an der der restlichen Innenwand zugewandten Fläche bevorzugt ein solches Profil auf, das eine definierte / zentrierte Anlage des Bügels auf/an der Innenwand ermöglicht ist.

In einer konkreten vorteilhaften Weiterbildung der vorliegenden Erfindung hat der erfindungsgemäße Siebkorb zwei sich gegenüberliegende, von einer Bodenplatte im Wesentlichen vertikal sich erstreckende, schmale Seitenwände (Stirnflächen) und zwei sich gegenüberliegende, von einer Bodenplatte im Wesentlichen vertikal sich erstreckende, breite Seitenwände (Längsflächen), die zusammen mit den zwei schmalen Seitenwänden und der Bodenplatte das maximale Behälter-Aufnahmevolumen definieren. An den schmalen Seitenwänden zugewandten Endbereichen der breiten Seitenwände sind jeweils ein Griffbügel über Schwenkgelenke gelagert, in welchen die Vorspannfedern untergebracht/integriert sind, welche die Griffbügel in deren aufgeschwenkte (Entnahme-)position vorspannen. Die Griffbügel sind derart geformt und so platziert, dass sie sich in deren eingeschwenkter (Verriegelungs-)position (im Wesentlichen konturengetreu) längs der jeweiligen zugeordneten schmalen Seitenwand erstrecken und diese hierbei abschnittsweise aussteifen und/oder abschnittsweise quasi als Seitenwandersatz ergänzen/komplettieren, d.h., einen wirksamen Flächenanteil der jeweiligen schmalen Seitenwand ausbilden. In/an den jeweils schmalen Seitenwänden sind die ausrückbaren Halte-/Freigabeelemente z.B. in Form eines federvorgespannten Bolzens oder einer federvorgespannten Schwenkkralle federangeordnet, welche in manuell unbetätigtem Zustand die Griffbügel in deren eingeschwenkten Verriegelungspositionen halten.

Eine weitere vorteilhafte/alternative Ausführungsform ist dadurch gekennzeichnet, dass das ausrückbare Halteelement als eine federvorgespannte verschiebbare Rastnase / Rastbolzen, etwa nach Art eines Schiebers, ausgestaltet ist. Ein Schieber eignet sich, um zuverlässig der Vorspannung des Griffs, welche ihn von der Verriegelungsposition in die Entnahmeposition treibt, entgegenzuwirken.

Alternativ zur verschiebbaren Rastnase ist das ausrückbare Halteelement als eine federvorgespannte verdrehbare (Dreh-)Scheibe ausgestaltet. Diese ermöglicht einem Anwender mittels der intuitiven Verdrehung um wenige Grad gegen eine Vorspannung, vorzugsweise um weniger als 10°, die Überführung von der Verriegelungsposition in die Entnahmeposition hervorzurufen.

Weiter alternativ zur Rastnase oder der Drehscheibe ist das ausrückbare Halteelement als ein verschwenkbares Klemmblech, etwa nach Art einer Blattfeder, ausgestaltet. Dies birgt den Vorteil, dass die erforderliche Vorspannung des Halteelements von dem Halteelement selbst verwirklicht ist, wodurch kein zusätzliches Federbauteil benötigt wird.

In einer weiteren vorteilhaften Ausgestaltung ist das aktuierbare Halte-/Freigabeelement als ein Push-to-open-Mechanismus ausgestaltet. Bei einem solchen Mechanismus fährt mittels eines sanften Drucks auf das Halte-/Freigabeelement der Griff von der Entriegelungsposition in die Entnahmeposition. Hierfür wird beim Überführen des Griffs in die Verriegelungsposition eine Feder des Halte-/Freigabeelements vorgespannt, welche sodann in jener vorgespannten Position gehalten ist, sodass ein unerwünschtes Wiederöffnen vermieden ist. Wird gegen den Griff in der Verriegelungsposition gedrückt, wird die zuvor gespannte Feder gelöst und ein Stift überführt den Griff in die Entnahmeposition.

Insbesondere vorteilhaft ist es weiterhin, wenn das ausrückbare Halteelement des Siebkorbs in der schmalen Behälterbreitenrichtung, das heißt in der Richtung, in der der Griff verläuft bzw. entlang der schmalen Stirnseite, im Wesentlichen mittig des Griffs angeordnet ist. So ist das Halteelement leicht zu erreichen und die von ihm ausgehende Kraft wirkt sich gleichmäßig auf den Griff aus.

In einer weiteren vorteilhaften Ausführungsform weist der Siebkorb neben dem Aufnahmebehälter und dem darin gelagerten Griff einen Deckel für den sicheren Transport des Siebkorbs auf. Der Deckel ist hierbei mittels der Betätigung eines Deckellösemechanismus von dem Aufnahmebehälter entnehmbar (d.h. wenn der Deckellösemechanismus betätigt wurde, wird ein Form- oder ein Kraftschluss gelöst, welcher ein Entnehmen des Deckels von dem Aufnahmebehälter ermöglicht), wobei die Betätigung des Deckellösemechanismus zugleich die Betätigung des ausrückbaren Halteelements auslöst. Dies vereinfacht die Handhabung des Siebkorbs weiter: So ist nur ein Deckellösemechanismus zu aktuieren, um erstens den Deckel entnehmen zu können zu zweitens die Griffe bereits in der Entnahmeposition vorzufinden.

In einer weiteren Ausführungsform mit dem Deckel könnte zwischen dem Deckellösemechanismus und dem ausrückbaren Halteelement ein Getriebe / ein Umwandlungsmechanismus angeordnet sein, das die aus der Betätigung / Aktuierung des Deckellösemechanismus resultierende Bewegung in eine das ausrückbare Halteelement betätigende Bewegung wandelt. Dieser zusätzliche Umwandlungsmechanismus stellt eine unmittelbare und folglich robuste Verbindung zwischen dem Deckel und dem ausrückbaren Halteelement her.

In einer weiteren vorteilhaften Ausgestaltung des erfindungsgemäßen Siebkorbs weist die Schenkelfeder eine degressive Kennlinie auf, sodass das Überführen von der Verriegelungsposition in die Entnahmeposition zu Beginn des Überführens schneller abläuft als zum Ende. Dies hat für einen Anwender den angenehmen Effekt, dass er nach der Betätigung des ausrückbaren Halteelements unmittelbar eine schnelle Bewegung des Griffs wahrnimmt, welche dann geordnet in die Entnahmeposition schließt. Hierbei ist es bevorzugt möglich, einen einstellbaren Dämpfungsmechanismus im Bereich des Griffs vorzusehen, über welchen sich die Bewegungskennlinie des Griffs anpassen lässt.

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen. Es zeigen:
- Fig. 1: eine perspektivische Ansicht eines erfindungsgemäßen Siebkorbs mit einem Aufnahmebehälter und einem Griff in einer Entnahmeposition sowie mit einem Deckel;
- Fig. 2: eine schematisch dargestellte Schenkelfeder (Bereich II aus Fig. 1) in der Verriegelungsposition;
- Fig. 3: die schematisch dargestellte Schenkelfeder aus Fig. 2 in der Überführung zur Entnahmeposition;
- Fig. 4: eine perspektivische Ansicht eines erfindungsgemäßen Siebkorbs mit Aufnahmebehälter und Griff in einer Verriegelungsposition;
- Fig. 5: eine perspektivische Ansicht eines erfindungsgemäßen Siebkorbs mit Aufnahmebehälter und Griff in der Verriegelungsposition gemäß einer weiteren Ausführungsform; und
- Fig. 6: eine weitere Ausgestaltung des Griffs.

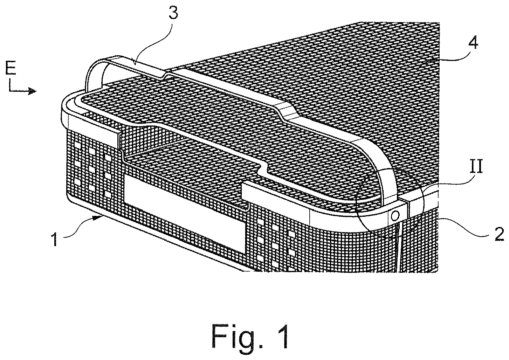

Fig. 1 stellt allgemein einen Siebkorb 1 dar, welcher sich vorliegend aus einem Aufnahmebehälter 2, zumindest einem Griff 3 und einem Deckel 4 zusammensetzt. Der Siebkorb 1 bzw. der daran gelagerte Griff 3 aus Fig. 1 befindet sich in der Entnahmeposition E, wie an dem hervorstehenden Griff 3 erkennbar ist.

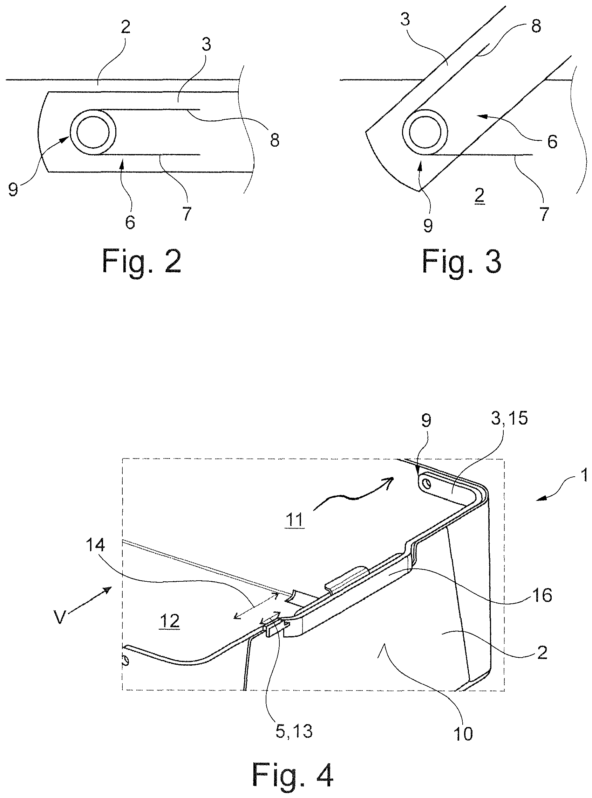

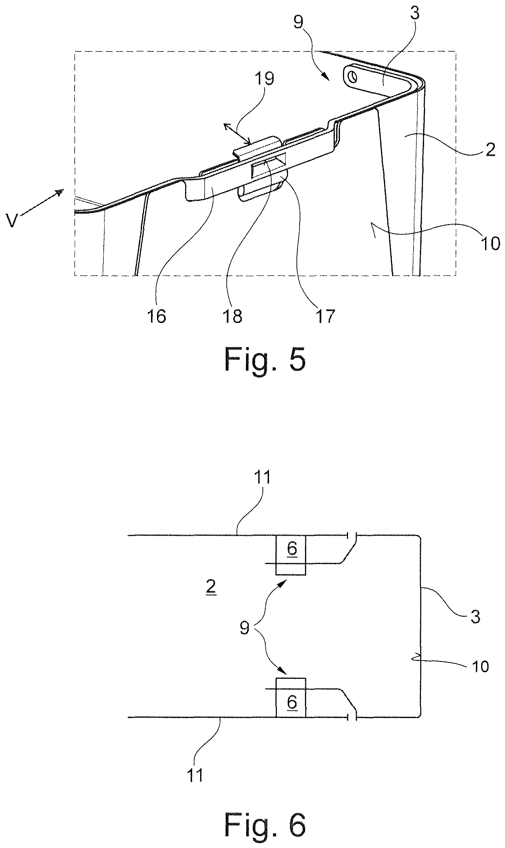

Gemäß der Erfindung wird die Entnahmeposition, d. h. das bevorzugt senkrechte Hervorstehen des Griffs 3 aus dem Aufnahmebehälter 2, nach einer entsprechenden Betätigung eines ausrückbaren Halteelements 5 (in Fig. 1 nicht dargestellt, vgl. Fign. 4 oder 5) automatisch/selbsttätig hervorgerufen. Hierfür ist in dem in Fig. 1 mit II dargestellten Bereich ein Federelement angeordnet, welches den Griff bzw. dessen Griffbügel 3 in die (aufgeschwenkte) (Entnahme-)position drängt.

Eine beispielhafte Ausführungsform des Federelements im Bereich II ist in Fig. 2 schematisch angedeutet. Hier ist zwischen dem Griff/Griffbügel 3 und dem Aufnahmebehälter 2 eine Schenkelfeder 6 angeordnet, die einen Fixschenkel 7 und einen Bewegungsschenkel 8 aufweist. Diese beiden Schenkel 7, 8 sind (wie bei einer Schenkelfeder üblich) gegeneinander über einen Windungsabschnitt vorgespannt. Jene Vorspannung erreicht ein Maximum, wenn, wie in Fig. 2 dargestellt, die beiden Schenkel parallel zueinander verlaufen.

Sobald das ausrückbare Halteelement 5 von einem Anwender betätigt wird, gibt dieses den Griffbügel 3 in seiner Verriegelungsposition frei, worauf die Vorspannung der Schenkelfeder 6 den Griff/Griffbügel 3 von einer Verriegelungsposition V (vgl. Fign. 4 und 5) in die Entnahmeposition E überführt. Während dieses Übergangs/Überführung nimmt der Winkel zwischen dem Fixschenkel 7 und dem Bewegungsschenkel 8 kontinuierlich zu (und die Federkraft folglich ab).

Wie etwa in Fig. 3 gezeigt, bleibt der Fixschenkel 7 in seiner Position, während der Bewegungsschenkel 8 von diesem wegwandert.

Die Schenkelfeder 6 ist beispielsweise im Bereich eines Griffgelenks 9 des erfindungsgemäßen Griffs angeordnet. Hierbei ist der Fixschenkel 7 mit dem Aufnahmebehälter 2 zu koppeln, während der Bewegungsschenkel 8 fest mit dem Griff/Griffbügel 3 verbunden ist, um diesen zuverlässig von der Verriegelungsposition V in die Entnahmeposition E zu überführen.

Die erfindungsgemäße Lösung ist nicht auf die nunmehr dargestellte Schenkelfeder beschränkt. Stattdessen können auch andere Federtypen, wie etwa Biegefedern, vorzugsweise Blattfedern, Spiralfeder oder wie etwa Torsionsfedern, vorzugsweise Stabfedern, Schraubenfedern, eingesetzt werden.

In Fig. 3 ist der Bewegungsschenkel 8 um rund 45° vom Fixschenkel 7 verdreht. In der endgültigen Entnahmeposition E ist der Winkel zwischen dem Bewegungsschenkel 8 und dem Fixschenkel 7 rund 90°. Dies bewirkt eine optimale Zugänglichkeit des Griffs 3 in der Entnahmeposition E. Durch eine bevorzugt degressive Kennlinie der Schenkelfeder 6 sind die ersten 45° schneller zurückgelegt als die zweiten 45°.

In Fig. 4 ist der Siebkorb 1 mit dem Aufnahmebehälter 2 und dem Griff/Griffbügel 3 in der Verriegelungsposition V dargestellt. Der Griff 3 ist bügelartig ausgeformt und verläuft komplementär zu einer Stirnfläche/schmale Seitenwand 10 des Aufnahmebehälters 2. Die Stirnfläche/schmale Seitenwand 10 bildet zusammen mit einer Seitenfläche/breiten Seitenwand 11 und einem Boden 12 des Aufnahmebehälters 2 dessen quaderförmige Grundform.

Der bügelartige Griff 3 schmiegt sich in Verriegelungsposition an die zugehörige Stirnfläche 10 unter Ausbildung eines zumindest abschnittsweisen Anlagekontakts an und ist in/an der Seitenfläche 11 durch das Griffgelenk 9 gelagert. So folgt eine optimale Raumausnutzung des Innenraums des Aufnahmebehälters 2.

In Fig. 4 ist das ausrückbare Halteelement 5 als ein in Richtung hin zu einer der Seitenflächen 11 bewegbarer Schieber 13 ausgebildet, der an einer Außenseite der Stirnfläche 10 gelagert ist. Der Schieber 13 ist federvorgespannt entlang der Wirklinie 14 bewegbar, die längs/parallel des in Verriegelungsposition befindlichen Griffbügels 3 ausgerichtet ist. Die entsprechende, für die Federvorspannung verantwortliche Feder ist in Fig. 4 nicht dargestellt kann aber eine einfache Schraubendruckfeder sein. Der Griff/Griffbügel 3 ist in einen Halteabschnitt 15 und einen Greifabschnitt 16 einteilbar, wobei sich der Halteabschnitt 15 an den beiden längsbeabstandeten Enden der Greifabschnitts 16 im Wesentlichen rechtwinklig erstreckt und an seinem freien Ende einen Wirkabschnitt (z.B. ein Durchsteckloch) hat, der mit dem Gelenk 9 schwenkgekoppelt ist. Des Weiteren ist im Greifabschnitt 16 des Griffbügels 3 eine Hinterschneidung ausgebildet/vorgesehen, die mit dem Schieber 13 für ein Halten des Griffbügels 3 in Verriegelungsposition entgegen der Federvorspannung der (Schenkel-)feder 6 in Wirkeingriff ist.

Sobald der Schieber 13 von dem Greifabschnitt 16 wegbewegt wird, löst sich der Formschluss zwischen dem Griffbügel 3 bzw. der daran ausgebildeten Hinterschneidung und dem Schieber 13, sodass die im Bereich des Griffgelenks 9 angeordnete Schenkelfeder 6 eine automatische Bewegung des Griffs in die Entnahmeposition E (vgl. Fig. 1) erreicht. Solange sich der Schieber 13 in dem Formschluss mit dem Griff 3 / der Hinterschneidung befindet, bleibt der Griff 3 in der Verriegelungsposition V aus Fig. 4. Ein einfaches Schieben des Schiebers 13 bewirkt also das Übergehen von Verriegelungsposition V in Entnahmeposition E.

Eine alternative Ausführungsform des manuell ausrückbaren Halteelements 5 ist in Fig. 5 dargestellt. Hier ist das ausrückbare Halteelement 5 als Klemmblech 17 ausgebildet. Dieses Klemmblech 17 bildet eine Blattfeder, die aus der Vorspannung des Blechs heraus mit dem Greifabschnitt 16 des Griffbügels 3 zusammenwirkt. Hierfür ist in dem Greifabschnitt 16 eine Ausbeulung oder Durchgriffsöffnung 18 angeordnet/ausgebildet, die einen Formschluss zwischen dem Klemmblech 17 und dem Greifabschnitt 16 ermöglicht.

Sobald das Klemmblech 17 entlang seiner Wirklinie 19 von dem Greifabschnitt 16 weggedrückt wird, löst sich der Formschluss zwischen dem Griffbügel 3 und dem Klemmblech 17, sodass die im Bereich/innerhalb des Griffgelenks 9 angeordnete Schenkelfeder 6 die automatische Bewegung des Griffs in die Entnahmeposition E (vgl. Fig. 1) erreicht. Solange sich das Klemmblech 17 in dem von der Ausbeulung 18 hervorgerufenen Formschluss mit dem Griff 3 befindet, bleibt der Griff 3 in der Verriegelungsposition V aus Fig. 5. Ein einfaches Drücken des Klemmblechs 17 bewirkt also das Übergehen von Verriegelungsposition V in Entnahmeposition E.

In Fig. 6 ist eine weitere Ausführungsform des erfindungsgemäßen Siebkorbs 1 dargestellt. Die Seitenwände 11 sind in der Draufsicht schematisch erkennbar. Über das Griffgelenk 9, bzw. die Griffgelenke, in denen jeweils eine Schenkelfeder 6 angeordnet ist, ist der Griff 3 mit dem Aufnahmebehälter 2 gekoppelt.

Gemäß der Ausführungsform aus Fig. 6 nimmt der Deckel 3 zumindest teilweise die Stirnfläche 11 des Siebkorbs 1 ein. Dies ist ermöglicht, indem der Deckel 3 nach dem Anlenken an dem Griffgelenk 9 sich vom Behälterinneren weg nach außen schwingt. Das von Gegenständen / Instrumenten zugängliche Innenvolumen des Aufnahmebehälters 2 ist auf diese Weise maximiert.

### Bezugszeichenliste

- 1: Siebkorb
- 2: Aufnahmebehälter
- 3: Griff
- 4: Deckel
- 5: Ausrückbares Halteelement
- 6: Schenkelfeder
- 7: Fixschenkel
- 8: Bewegungsschenkel
- 9: Griffgelenk
- 10: Stirnfläche
- 11: Seitenfläche
- 12: Boden
- 13: Schieber
- 14: Wirklinie des Schiebers
- 15: Halteabschnitt
- 16: Greifabschnitt
- 17: Klemmblech
- 18: Ausbeulung
- 19: Wirklinie des Klemmblechs
- E: Entnahmeposition
- V: Verriegelungsposition

## Patentansprüche

1. Siebkorb (1) zur Aufnahme zu desinfizierender oder sterilisierender medizinischer Gegenstände, mit einem Aufnahmebehälter (2) und zumindest einem darin oder daran gelagerten Griff (3), der in einer Entnahmeposition (E) aus/von dem Aufnahmebehälter (2) greifbar hervorsteht, um ein Tragen des Siebkorbs (1) zu ermöglichen, und der in einer Verriegelungsposition (V) in/an dem Aufnahmebehälter (2) anliegend angeordnet ist,
**dadurch gekennzeichnet, dass**
der Griff (3) durch einen automatisch wirkenden Antriebsmechanismus in seine Entnahmeposition (E) selbsttätig, vorzugsweise federvorgespannt, bewegbar ist und durch einen Halte-/Freigabemechanismus in seiner Verriegelungsposition (V) haltbar ist, der hierfür ein direkt oder indirekt manuell ausrückbares sowie vorzugswiese von der Siebkorbaußenseite erreichbares und aktuierbares Halte-/Freigabeelement (5) hat.

2. Siebkorb (1) nach Anspruch 1, **dadurch gekennzeichnet, dass** zumindest eine Schenkelfeder (6) als Antriebsmechanismus für die Überführung des Griffs (3) von der Verriegelungsposition (V) in die Entnahmeposition (E) verantwortlich ist.

3. Siebkorb (1) nach einem der vorstehenden Ansprüche, **dadurch gekennzeichnet, dass** der Aufnahmebehälter (2) einen im Wesentlichen rechteckigen Boden hat, längs dessen Umfangs sich schmale und breite Seitenwände (10, 11) erstrecken, wobei längs der schmalen Seitenwände (10) jeweils ein bügelartiger Griff (3) angeordnet ist, der sich über die gesamte schmale Seitenwand (10) erstreckt und an den sich gegenüberliegenden breiten Seitenwänden schwenkbar gelagert ist.

4. Siebkorb (1) nach Anspruch 3, **dadurch gekennzeichnet, dass** die bügelartigen Griffe (3) in der Verriegelungsposition (V), vorzugsweise zumindest abschnittsweise annährend schlitzfrei, entlang der schmalen Seitenwände (10) angeordnet sind, sodass das von Gegenständen erreichbare Volumen des Innenraums des Aufnahmebehälters (2) nur um das Volumen des jeweiligen bügelartigen Griffs (3) verkleinert ist.

5. Siebkorb (1) nach Anspruch 3 oder 4, **dadurch gekennzeichnet, dass** zumindest die schmalen Seitenwände (10) jeweils an ihrer dem Boden abgewandten oberen Stirnseite eine Einkerbung oder Ausnehmung aufweisen, wobei der bügelartige Griff (3) in seinem Längsmittenabschnitt nach außen gebogen ist, derart, dass der nach außen gebogene Griffbügelabschnitt in die Einkerbung oder Ausnehmung ragt und somit die schmale Seitenwand (10) im Bereich ihrer Einkerbung oder Ausnehmung im Wesentlichen bündig ergänzt, wobei der Griffbügel (3) in der Verriegelungsposition (V) einen Teil der schmalen Seitenwand (10) selbst ausformt.

6. Siebkorb (1) nach einem der vorstehenden Ansprüche, **dadurch gekennzeichnet, dass** das ausrückbare Halteelement (5) als eine federvorgespannte, verschiebbare Rastnase, etwa nach Art eines Schiebers (13), oder als eine federvorgespannte verdrehbare Drehscheibe oder als ein verschwenkbares Klemmblech (17) ausgestaltet ist.

7. Siebkorb (1) nach einem der vorstehenden Ansprüche, **dadurch gekennzeichnet, dass** das aktuierbare Halte-/Freigabeelement als ein Push-to-open-Mechanismus ausgestaltet ist.

8. Siebkorb (1) nach einem der vorstehenden Ansprüche, **dadurch gekennzeichnet, dass** das ausrückbare Halteelement (6) in der Behälterbreitenrichtung im Wesentlichen mittig des Griffbügels (3) angeordnet ist.

9. Siebkorb (1) nach einem der vorstehenden Ansprüche, **dadurch gekennzeichnet, dass** der Siebkorb (1) einen Deckel (3) aufweist, welcher mittels der Betätigung eines Deckellösemechanismus von dem Aufnahmebehälter (2) entnehmbar ist, wobei der Deckellösemechanismus mit dem Halte-/Freigabemechanismus gekoppelt oder wirkverbunden ist, derart, dass die Betätigung des Deckellösemechanismus zugleich die Betätigung des ausrückbaren Halteelements (6) auslöst.

10. Siebkorb (1) nach Anspruch 9, **dadurch gekennzeichnet, dass** zwischen dem Deckellösemechanismus und dem ausrückbaren Halteelement (6) ein Getriebe angeordnet ist, das die aus der Betätigung des Deckellösemechanismus resultierende Bewegung in eine ausrückende Bewegung des ausrückbaren Halteelements (6) wandelt.

11. Siebkorb (1) nach einem der Ansprüche 2 bis 10, **dadurch gekennzeichnet, dass** die Schenkelfeder (6) eine degressive Kennlinie aufweist, sodass das Überführen von der Verriegelungsposition (V) in die Entnahmeposition (E) zu Beginn des Überführens schneller abläuft als zum Ende.

## Claims

1. Sieve basket (1) for receiving medical articles to be disinfected or sterilized, having a receiving container (2) and at least one handle (3) mounted therein or thereon, which in a removal position (E) projects out of/from the receiving container (2) in a manner such that it can be grasped in order to enable the sieve basket (1) to be carried, and which in a locking position (V) is arranged in/abutting on the receiving container (2),
**characterized in that**
the handle (3) can be moved automatically, preferably in a spring-preloaded manner, into its removal position (E) by an automatically operating driving mechanism and can be held in its locking position (V) by a holding/release mechanism, which has for this purpose a holding/release element (5) which can be manually disengaged directly or indirectly and can preferably be reached and actuated from the outside of the basket.

2. Sieve basket (1) according to claim 1, **characterized in that** at least one leg spring (6) is responsible as driving mechanism for transferring the handle (3) from the locking position (V) to the removal position (E).

3. Sieve basket (1) according to one of the preceding claims, **characterized in that** the receiving container (2) has a substantially rectangular bottom, along the circumference of which narrow and wide side walls (10, 11) extend, wherein along each of the narrow side walls (10) a bracket-like handle (3) is arranged, which extends over the entire narrow side wall (10) and is pivotably mounted on the opposite wide side walls.

4. Sieve basket (1) according to claim 3, **characterized in that** the bracket-like handles (3) are arranged in the locking position (V), preferably at least in sections virtually without slots, along the narrow side walls (10), so that the volume of the interior space of the receiving container (2) accessible by objects is reduced only by the volume of the respective bracket-like handle (3).

5. Sieve basket (1) according to claim 3 or 4, **characterized in that** at least the narrow side walls (10) each have a notch or recess at their upper end side facing away from the ground, wherein the bracket-like handle (3) is curved outwards in its longitudinal center portion, in such a way that the handle bracket portion, which is curved outwards, projects into the notch or recess and thus substantially complements the narrow side wall (10) in a flush manner in the region of its notch or recess, wherein the handle bracket (3) itself forms part of the narrow side wall (10) in the locking position (V).

6. Sieve basket (1) according to one of the preceding claims, **characterized in that** the disengageable holding element (5) is designed as a spring-preloaded, displaceable latching nose, for instance in the manner of a slider (13), or as a spring-preloaded, rotatable disk or as a pivotable clamping plate (17).

7. Sieve basket (1) according to one of the preceding claims, **characterized in that** the actuatable holding/release element is designed as a push-to-open mechanism.

8. Sieve basket (1) according to one of the preceding claims, **characterized in that** the disengageable holding element (6) is arranged substantially centrally of the handle bracket (3) in the container width direction.

9. Sieve basket (1) according to one of the preceding claims, **characterized in that** the sieve basket (1) has a cover (3) which can be removed from the receiving container (2) by means of the actuation of a cover-release mechanism, wherein the cover-release mechanism is coupled or operatively connected to the holding/release mechanism in such a way that the actuation of the cover-release mechanism simultaneously triggers the actuation of the releasable holding element (6).

10. Sieve basket (1) according to claim 9, **characterized in that** a gear is arranged between the cover-release mechanism and the disengageable holding element (6) which converts the movement resulting from the actuation of the cover-release mechanism into a disengaging movement of the disengageable holding element (6).

11. Sieve basket (1) according to one of claims 2 to 10, **characterized in that** the leg spring (6) has a degressive characteristic curve, so that the transfer from the locking position (V) to the removal position (E) is faster at the beginning of the transfer than at the end.

## Revendications

1. Panier crible (1) pour la réception d'objets médicaux à désinfecter ou à stériliser, avec un récipient de réception (2) et au moins une poignée (3) logée dedans ou dessus, qui fait saillie dans une position de prélèvement (E) de manière saisissable depuis le/du récipient de réception (2), pour permettre de porter le panier crible (1), et qui est agencée dans une position de verrouillage (V) en appui dans/sur le récipient de réception (2),
**caractérisé en ce que**
la poignée (3) est déplaçable automatiquement, de préférence précontrainte par ressort, dans sa position de prélèvement (E) par un mécanisme d'entraînement à action automatique et peut être retenue dans sa position de verrouillage (V) par un mécanisme de retenue/libération, qui a pour ce faire un élément de retenue/libération (5) débrayable directement ou indirectement manuellement ainsi que de préférence accessible depuis le côté extérieur du panier crible et actionnable.

2. Panier crible (1) selon la revendication 1, **caractérisé en ce qu'**au moins un ressort à branches (6) est responsable en tant que mécanisme d'entraînement du passage de la poignée (3) de la position de verrouillage (V) à la position de prélèvement (E).

3. Panier crible (1) selon l'une quelconque des revendications précédentes,
**caractérisé en ce que** le récipient de réception (2) a un fond sensiblement rectangulaire, le long de la périphérie duquel des parois latérales étroites et larges (10, 11) s'étendent, dans lequel le long des parois latérales étroites (10) respectivement une poignée de type étrier (3) est agencée, qui s'étend sur l'ensemble de la paroi latérale étroite (10) et est logée de manière pivotante au niveau des parois latérales larges se faisant face.

4. Panier crible (1) selon la revendication 3, **caractérisé en ce que** les poignées (3) de type étrier sont agencées dans la position de verrouillage (V), de préférence au moins par section approximativement sans fente, le long des parois latérales étroites (10), de sorte que le volume pouvant être atteint par les objets de l'espace intérieur du récipient de réception (2) est seulement réduit du volume de la poignée (3) de type étrier respective.

5. Panier crible (1) selon la revendication 3 ou 4, **caractérisé en ce qu'**au moins les parois latérales étroites (10) présentent respectivement au niveau de leur côté frontal supérieur opposé au fond une encoche ou un évidement, dans lequel la poignée (3) de type étrier est courbée vers l'extérieur dans sa section médiane longitudinale, de sorte que la section d'étrier de poignée courbée vers l'extérieur pénètre dans l'encoche ou l'évidement et complète ainsi sensiblement en affleurement la paroi latérale étroite (10) dans la zone de son encoche ou évidement, dans lequel l'étrier de poignée (3) forme lui-même dans la position de verrouillage (V) une partie de la paroi latérale étroite (10).

6. Panier crible (1) selon l'une quelconque des revendications précédentes,
**caractérisé en ce que** l'élément de retenue (5) débrayable est configuré en tant qu'ergot d'encliquetage coulissant, précontraint par ressort, par exemple à la manière d'un coulisseau (13), ou en tant que disque rotatif pouvant tourner précontraint par ressort ou en tant que tôle de serrage (17) pivotante.

7. Panier crible (1) selon l'une quelconque des revendications précédentes,
**caractérisé en ce que** l'élément de retenue/libération actionnable est configuré en tant que mécanisme push-to-open.

8. Panier crible (1) selon l'une quelconque des revendications précédentes,
**caractérisé en ce que** l'élément de retenue (6) débrayable est agencé dans le sens de la largeur du récipient sensiblement au milieu de l'étrier de poignée (3).

9. Panier crible (1) selon l'une quelconque des revendications précédentes,
**caractérisé en ce que** le panier crible (1) présente un couvercle (3), lequel peut être enlevé du récipient de réception (2) par l'actionnement d'un mécanisme de libération de couvercle, dans lequel le mécanisme de libération de couvercle est couplé ou en liaison active avec le mécanisme de retenue/libération, de sorte que l'actionnement du mécanisme de libération de couvercle déclenche en même temps l'actionnement de l'élément de retenue (6) débrayable.

10. Panier crible (1) selon la revendication 9, **caractérisé en ce qu'**une transmission est agencée entre le mécanisme de libération de couvercle et l'élément de retenue (6) débrayable, qui convertit le mouvement résultant de l'actionnement du mécanisme de libération de couvercle en un mouvement de débrayage de l'élément de retenue (6) débrayable.

11. Panier crible (1) selon l'une quelconque des revendications 2 à 10, **caractérisé en ce que** le ressort à branches (6) présente une courbe caractéristique dégressive, de sorte que le passage de la position de verrouillage (V) à la position de prélèvement (E) est plus rapide au début du passage qu'à la fin.
